# EUROPÄISCHE PATENTSCHRIFT

(11) **EP 0 575 695 B1**
(45) Veröffentlichungstag und Bekanntmachung des Hinweises auf die Patenterteilung: **17.06.1998**
(21) Anmeldenummer: 93102747.8
(22) Anmeldetag: 22.02.1993
(51) Int. Cl.: C12N 11/08, C02F 3/10, C02F 3/30

(54) **Flächiges, biologisch abbaubares Trägermaterial für Denitrifikanten in biologisch betriebenen Klärstufen**
Flat, biodegradable carrier material for denitrificants in biological sewage plants
Matériau support plan pour des dénitrificants dans des stations d'épuration

(30) Priorität: 25.06.1992 DE 4220795
(43) Veröffentlichungstag der Anmeldung: 29.12.1993
(73) Patentinhaber: Firma Carl Freudenberg, 69469 Weinheim (DE)
(72) Erfinder: Groten, Robert, Dr., W-6940 Weinheim/Steinklingen (DE); Heidecke, Gerhard, Dr., W-6140 Bensheim 1 (DE); Mannsbart, Thomas, W-6803 Edingen-Neckarhausen (DE); Siekermann, Volker, Dr., W-6149 Fürth (DE)

(56) Entgegenhaltungen:
- DE-A- 3 410 412
- FR-A- 2 592 597
- UMWELT, Bd.20, Nr.11, November 1990, DE DÜSSELDORF Seiten 593 - 595 WOLF-RÜDIGER MÜLLER 'ABWASSER MIT ZERKLEINERTEM KUNSTSTOFF REINIGEN'
- GWF WASSER ABWASSER, Bd.134, Nr.1, Januar 1993, MUNCHEN DE Seiten 80 - 84 STEPHAN WAIS ET AL. 'EINSATZ EINER STARTERCULTUR ZUR DENITRIFIKATION IN FESTBETTREAKTOREN MIT POLY-(3-HYDROXYBUTTE RSÄURE-CO-3-HYDROXYVALERIANSÄURE) ALS FÜLLMATERIAL'

## Beschreibung

Die Erfindung befaßt sich mit einem flächigen, biologisch abbaubaren Trägermaterial, welches spontan aufwachsende Denitrifikantenpopulationen, also spezielle Mikroorganismen und Bakterien, die Nitrat abbauen, aufzunehmen in der Lage ist.

In dem Beitrag "Abwasser mit zerkleinertem Kunststoff reinigen" von Wolf-Rüdiger Müller, Umwelt, Band 20 (1990), wird ein biologisch abbaubares Trägermaterial aus Poly-β-Hydroxybuttersäure/Poly-β-Hydroxyvaleriansäure (PHB/PHV) zur biologischen Nitratelimination (Denitrifikation) beschrieben. Dieses Trägermaterial wird biotechnisch in Fermentern durch eine Stoffwechselreaktion von Bakterien gewonnen und liegt in Form einer Folie vor. Die Besiedlung mit Denitrifikantenpopulationen und die Möglichkeit einer Denitrifikation ohne Zugabe weiterer externer, organischer Wasserstoff- und Kohlenstoff-Donatoren werden untersucht. Über den NOₓ-Gehalt nach der Nitratelimination wird nichts ausgesagt.

Die Folie selbst ist als Wasserstoff-und Kohlenstoff-Quelle mikrobiell nutzbar. Der dadurch mögliche Verzicht auf Methanol als Donator ist für die Trinkwasseraufbereitung aus toxischen Gründen von hohem Wert.

Mit diesem Trägermaterial kann in einem Silo oder Becken eine der Filtrations- und der Nitrifikationsstufe nachgeschaltete Denitrifikation bei der biologischen Abwasserreinigung von Ab- und Grundwässern betrieben werden. Diese Verfahrenstechnik beinhaltet einen Zwischenaufenthalt bereits verwendeten Trägermaterials in einem Silo, worin neue Denitrifkanten-Populationen auf den Träger aufwachsen.

Die Nachteile dieses Verfahrens sind die hohen Kosten, die durch die Biosynthese des Trägermaterials verursacht werden und daher dessen umfänglicher Verwendung entgegenstehen. Außerdem sind nur begrenzte Mengen an biotechnologisch über Fermenter hergestelltem PHB/PHV pro Jahr verfügbar. PHB/PHV-Systeme sind auch nur zu Folienträgern verarbeitbar, deren geringe Oberfläche eine begrenzte Aufnahmekapazität für Denitrifikanten besitzt und die nicht vergrößerbar ist.

Aufgabe der vorliegenden Erfindung ist es, ein preiswertes und in größeren Mengen herstellbares, leicht zu handhabendes Flächengebilde als Trägermaterial für die genannten Denitrifikanten anzugeben, welches noch effektiver als alleiniger Kohlenstoff- und Wasserstoff-Donator wirkt. Es soll schadstofffrei auch in nitratbelasteten Trinkwasseraufbereitungsanlagen einsetzbar sein und eine Denitrifikationsleistung von mindestens 45% NOₓ - N₂ ermöglichen (bei vorgeschalteter Nitrifizierung). Im Verhältnis zum Gewicht soll eine größere besiedelbare Oberfläche zur Verfügung stehen, als sie eine Folie bietet.

Die Lösung dieser Aufgabe besteht in der Bereitstellung eines Trägermaterials aus Vliesstoff mit den kennzeichnenden Merkmalen des ersten Patentanspruchs. Der Vliesstoff ist zur Gänze biologisch abbaubar und besteht zu mindestens 50 Gew.-% aus Poly-ε-Caprolacton-Spinnfilamenten, wobei gegebenenfalls der zweite Polymermischungs-Bestandteil biologisch abbaubares Polyhydroxybutyrat, Polyhydroxybutyrat-Hydroxyvalerat-Copolymer, ein Polylactid oder ein Polyesterurethan ist.

Der Vliesstoff besitzt ein Flächengewicht von 10 bis 1500 g/m². Seine Filamente haften bindemittelfrei an ihren Kreuzungspunkten aneinander aufgrund thermischer Verschweissung und bestimmen so die innere Festigkeit. Zweckmäßig sollen die Filamente möglichst dünn gewählt werden, um die für Denitrifikanten zur Verfügung stehende, besiedelbare Oberfläche groß zu halten. Grundsätzlich sind jedoch alle technisch herstellbaren Filamentdurchmesser für die vorliegende Erfindung geeignet.

Als Denitrifikanten-Typen eignen sich beispielsweise Flavo- und Agrobakterien oder Pseudomonaden.

Die für die jeweilige Klärstufe benötigte Menge an Vliesstoff muß durch Vorversuche ermittelt werden. Gesucht ist dabei das Optimum an Denitrifikationsleistung, bezogen auf die vorherrschende Durchströmungsmenge an Abwasser durch den Vliesstoff pro Fläche und Zeit.

Enthält ein Abwasser zum Beispiel 15 bis 25 mg NOₓ-N/l, entsprechend 65 bis 110 mg NOₓ/l, und geht man von einem analytisch ermittelten Verbrauch an Denitrifikantenfläche von 0,008 m² pro Monat sowie einem Wasserdurchfluß von 0,25 m³ pro Monat aus, so ergibt sich bei der Division der Oberfläche durch den Durchfluß ein Wert von 0,032 m² Vliesstoff pro m³ Wasser, um eine optimale Klärwirkung zu erhalten.

Werte dieser Größenordnung gelten ausschließlich für die oben angegebenen Konzentrationsbereiche an NOₓ. Zur Trinkwasseraufbereitung ist mit einem niedrigeren Wert zu rechnen, da der Denitrifikanten-Verbrauch geringer ist.

Poly-ε-Caprolacton ist bekanntermaßen leicht aus der Schmelze in beliebigen Filamentlängen spinnbar und in der Natur mit Hilfe von im Boden vorkommenden Organismen biologisch abbaubar. In der verwendeten Filamentform hat es ein Molekulargewicht von 20000 bis 70000.

Die Herstellung des Vliesstoffs erfolgt nach bekannter Technologie in der Weise, daß Poly-ε-Caprolacton oder das biologisch abbaubare Polyestergemisch auf 150 bis 220°C aufgeschmolzen, mittels Pumpen Düsen zugeführt und durch diese zu Filamenten versponnen wird. Diese Filamente werden durch temperierte Luft verstreckt und abgekühlt sowie dann zu einem Spinnvlies abgelegt.

Ein zusätzlicher Verfestigungsschritt ist nicht notwendig: Durch einfache Optimierung der Schmelz- und der Verstrekkungsluft-Temperatur kann erreicht werden, daß zum Zeitpunkt des Ablegens der frisch gesponnenen Filamente das Polymer sich noch im Zustand nicht abgeschlossener Kristallisation befindet, was zusammen mit der noch genügend hohen Oberflächentemperatur der Filamente zu einer solchen Klebrigkeit führt, daß eine thermoplastische Verschweißung an den Filament-Kreuzungspunkten von selbst auftritt.

Die Grenzen des Molekulargewichts sind dadurch gegeben, daß bei kleineren Werten die Masse zu wachsartig ist, um noch verspinnbar zu sein. Bei größeren Molekulargewichten ist das Polycaprolacton zu spröde.

Der erfindungsgemäße Vliesstoff kann zur Gänze aus Poly-ε-Caprolacton-Filamenten bestehen, mindestens jedoch zu 50 Gew.-%, wobei die zweite Polyesterkomponente dann einer der eingangs genannten biologisch abbaubaren Polyester ist. Diese Zwei-Komponentensysteme sind noch besser spinnbar und selbstverfestigend im Vliesstoff, welcher vollständig biologisch abbaubar bleibt.

Die Werkstoffe dieser zweiten Polyester-Komponenten sind zwar auch in reiner Form biologisch abbaubar, jedoch dann nicht oder nur mit großem technischen Aufwand aus ihrer Schmelze zu Filamenten spinnbar. Erst ihre Kombination mit Poly-ε-Caprolacton im Gemisch macht sie für konventionelle Schmelzspinnverfahren geeignet.

Die erfindungsgemäßen Träger-Spinnvliesstoffe besitzen eine Dehnbarkeit von mindestens 50% und sind permanent hydrophil.

Die Beladung mit den biologisch wirksamen Mikroorganismen geschieht durch spontanes Aufwachsen. Dabei siedeln sich überraschenderweise native Denitrifikantenpopulationen auch unter anaeroben Bedingungen an das Trägermaterial an. Man braucht also zu diesem Zwecke lediglich den Vliesstoff in das Denitrifikationssilo oder -becken einzubringen und das zu denitrifizierende Wasser hindurchzuleiten.

Es ist jedoch in Hinblick auf ein schnelleres Aufwachsen zweckmäßig, einmal besiedelte Vliestoffträger mit neuen, unbesiedelten kurz vor dem Verbrauch zusammenzubringen.

Die Träger weisen bei vollzogener Beladung mehrere Schichten von Denitrifikanten auf. Die technische Anwendung derartiger Träger erfolgt in der Weise, daß die Vliesstoffe jeweils durch Spannrahmen gehalten werden, welche durch ihr Gewicht die Träger unter Wasser halten. Zweckmäßig werden mehrere Spannrahmen hintereinander aufgereiht. Sie können ebenfalls aus Poly-ε-Caprolacton bestehen.

Die erfindungsgemäßen Träger sind wegen ihrer fehlenden Toxizität auch in Trinkwasseraufbereitungsanlagen zur Denitrifizierung einsetzbar. Im Vergleich zu PHB/PHV-Folien weisen sie eine im Verhältnis zur Oberfläche mindestens um den Faktor 2 raschere Denitrifikationsgeschwindigkeit (NOₓ→N₂) auf. Schwach nitratbelastete Wässer werden zu nahezu 100% denitrifiziert, dies alles als Folge der höheren Populationsdichte auf den Vliesstoffoberflächen.

### Beispiel 1

### Herstellung eines Polycaprolacton-Vliesstoffs

Poly-ε-Caprolacton mit einem Schmelzpunkt um 60°C sowie einem melt flow index von 10 g/10 min bei 130°C/2,16 kg wird bei einer Extrudertemperatur von 185°C aufgeschmolzen. Die Massetemperatur der Polymerschmelze beträgt 203°C. Die zur Verstreckung der aus den Spinndüsen austretenden Polymerschmelze benötigte Luft hat eine Temperatur von 50°C.

Die verstreckten Endlosfilamente werden auf einem Siebband aufgefangen und verbinden sich an ihren Kreuzungspunkten ohne weitere Verfestigung. Das Flächengewicht des Spinnvliesstoffs beträgt 22 g/m².

Der so hergestellte Spinnvliesstoff wurde für den Technikumsversuch als lose Vliesstoffbahn in sechs Schlaufen, jede in Beckenbreite, in das nachgeschaltete Denitrifikationsbecken eingelegt. Den Rahmen bildeten die gleichen Filamente wie diejenigen des Vliestoffs selbst; dabei wurde einfach der Rahmenbereich durch Anschmelzen der dort vorhandenen Fasern versteift. Die Anzahl von sechs Schlaufen wurde in Vorversuchen als optimal für die Denitrifizierung ermittelt.

### Beladung des Spinnvliesstoffs

Die in das Becken der Trink- und Abwasseraufbereitungsanlage eingebrachten Vliesstoffoberflächen wurden auf natürliche Weise mit denitrifizierenden Mikroorganismen aus dem Grundbeziehungsweise Abwasser besiedelt. Als sessile Spezies, die das angebotene, biologisch abbaubare Vliesstoffsubstrat beziehungsweise dessen Spaltprodukte am besten verstoffwechseln konnten, wurden nach der Adaptionsphase als beherrschende Denitrifikantengruppen Pseudomonaden und Bazillen gefunden:

Das Aufwachsen wurde durch die Parameter Temperatur, pH-Wert, Sauerstoffgehalt nach Nitrifikation und Verweilzeit des zu behandelnden Wassers beeinflußt: 30°C, pH 8,0, 0,5 mg O₂/l, 3 h.

Dieses Trägermaterial wurde in folgender Weise in einer Kläranlage eingesetzt:
1. Vliestoffsilo im Technikumsmaßstab
Abwasser einer Industriekläranlage wurde nach Fällung und Flockung eingesetzt. Die Abmessungen der Anlagen und die Volumenströme entsprachen den Daten der Kläranlage im Verhältnis 1:1000000.
Vor dem Zuführen des Abwassers in die Nitrifikationsstufe der Technikumsanlage wurde durch Sedimentation der entstehende Chemieschlamm entfernt.
Nach der Nitrifikation wurde der Bioschlamm in einem Absetzbecken sedimentiert und in die Nitrifikationsstufe zurückgeführt. Die Klarphase durchströmte die Denitrifikationsstufe von unten nach oben. Verwendet wurde ein Plexiglassilo mit Festbett aus Poly-ε-Caprolacton-Granulat.
Das Festbett wurde zu Beginn des Versuchs mit Denitrifikanten aus einer parallel dazu betriebenen Laborkläranlage angeimpft.
a) Variante mit nachgeschalteter Nitrifikation:
   Bei diesem Versuch wurde mittels der vorher beschriebenen Technikumsanlage der Wirkungsgrad der Denitrifikationsstufe ermittelt und mit den Werten der parallel dazu betriebenen Technikumsanlage mit vorgeschalteter Denitrifikation verglichen. Hierbei zeigten sich schon nach einer Adaptionszeit von weniger als 3 Wochen vergleichbare Denitrifikationsraten.
b) Variante mit nachgeschalteter Denitrifikation und geringer NOₓ -Konzentration:
   Dieser Versuch diente dazu zu prüfen, ob eine verminderte Nitrifikationsleistung durch Hemmung der Nitrifikanten die Denitrifikantenpopulation in ihrer Abbauleistung beeinträchtigt. Eine Beeinflussung konnte nicht festgestellt werden. Die angebotenen Nitrat- und Nitritkonzentrationen wurden fast vollständig abgebaut.

2. Vliesstoffsilo im Abwasser/Realbedingungen (mit vorgeschalteter Nitrifikation):
Die Anlagenkonzeption wurde im wesentlichen beibehalten. Es wurde ein offenes Becken, passend zur Geometrie des Kläranlagenbeckens, eingesetzt. Der Spinnvliesstoff wurde als loses Vliesstoffband in sechs Schlaufen in das Denitrifikationsbecken eingebracht. Der Zulauf erfolgte an der Oberkante der Stirnseite, der Ablauf unten an der gegenüberliegenden Seite des Beckens.
Mittels titrimetrischer und ionenchromatographischer Messungen wurde wöchentlich die Stickstoffbilanz (NH₄⁺ ,KJ-N,NOₓ) im Zulauf und Ablauf der Denitrifikationsstufe ermittelt und somit die Denitrifikationsrate NOₓ zu N₂ bestimmt:

| | | |
|---|---|---|
| Versuch 1., | Variante a) : | 40% |
| | Variante b) : | über 80% |
| Versuch 2.: | | über 70% |

Unter gleichen Bedingungen ergaben sich bei gleicher eingesetzter Werkstoffmenge für PHB/PHV-Folien, 100 µm dick, monoschicht-beladen, unter Realbedingungen Denitrifikationsraten unter 10%.

### Beispiel 2:

### Herstellung eines Polycaprolacton-Polyhydroxybutyrat/Hydroxyvalerat-Spinnvliestoffs

Eine Polymermischung aus 80 Gew.-% Poly-ε-Caprolacton und 20 Gew.-% Polyhydroxybutyrat/Hydroxyvalerat-Copolymer mit einem melt flow index von 34 g/10 min bei 190°C/216 kg wird bei 182°C aufgeschmolzen. Die aus den Spinndüsen austretende Polymerschmelze wird mit Luft verstreckt, deren Temperatur um 40°C liegt. Die verstreckten Endlosfilamente werden auf einem Transportband aufgefangen, und die Fasern verbinden sich ohne externe Verfestigung. Das Flächengewicht des Vliesstoffs beträgt 23 g/m². Seine Beladung erfolgt wie in Beispiel 1, ebenso die versuchsweise Anwendung.

Folgende Ergebnisse wurden erzielt:

| | | |
|---|---|---|
| Versuch 1. | a) | 35% |
| | b) | 75% |
| 2. | | 65% |

## Patentansprüche

1. Flächiges, biologisch abbaubares Trägermaterial für biologisch betriebene Klärstufen, welches in sauerstoffarmen Wässern als Kohlenstoff- und Wasserstoff-Quelle wirkt und mit spontan aufwachsenden Denitrifikantenpopulationen in einem Ausmaß von mindestens einer vollflächigen Schichtbedeckung beladen ist, gekennzeichnet durch einen Spinnvliesstoff mit einem Flächengewicht von 10 bis 1500 g/m² und mit einer vorherbestimmten wirksamen Fläche, welche ein Optimum an Denitrifikationsleistung in der jeweiligen Klärstufe bei vorgegebener Durchströmungsmenge pro Fläche und Zeit ergibt, bestehend zu mindestens 50 Gew.-% aus Poly-ε-Caprolacton-Endlosfilamenten, wobei das Polycaprolacton im Mittel ein Molekulargewicht von 20000 bis 70000 aufweist und wobei die Einzelfilamente bindemittelfrei an ihren Kreuzungspunkten aneinander haften.

2. Trägermaterial nach Anspruch 1, dadurch gekennzeichnet, daß die Endlosfilamente zur Gänze aus Poly-ε-Caprolacton bestehen.

3. Trägermaterial nach Anspruch 1, dadurch gekennzeichnet, daß die Endlosfilamente aus einer zweikomponentigen polymeren Mischung bestehen, wobei die eine Komponente das Polycaprolacton, die andere biologisch abbaubares Polyhydroxybutyrat-Hydroxyvalerat-Copolymer, ein Polylactid oder ein Polyesterurethan ist.

## Claims

1. A flat, biodegradable support material for biologically acting purification stages, which material acts as carbon and hydrogen source in oxygen-poor waters and is loaded with spontaneously growing denitrification populations to an extent of at least one full-surface layer covering, characterized by a spunbonded nonwoven having a weight per unit area of from 10 to 1500 g/m² and having a predetermined active area which gives an optimum denitrification performance in the respective purification stage at a predetermined flow rate per unit area and time, comprising at least 50% by weight of poly-ε-caprolactone continuous filaments, the polycaprolactone having a mean molecular weight of from 20,000 to 70,000 and the individual filaments adhering to one another without binder at their crossing points.

2. A support material according to claim 1, characterized in that the continuous filaments consist entirely of poly-ε-caprolactone.

3. A support material according to claim 1, characterized in that the continuous filaments consist of a two-component polymer mixture, the one component being the polycaprolactone, the other being biodegradable poly(hydroxybutyrate-hydroxyvalerate) copolymer, a polylactide or a polyesterurethane.

## Revendications

1. Matériau support plan biodégradable pour des étages d'épuration biologique, qui agit comme source de carbone et d'hydrogène dans des eaux pauvres en oxygène et qui est chargé de populations de bactéries dénitrifiantes croissant de manière spontanée en au moins une couche de recouvrement complète, caractérisé par un non-tissé en filaments continus avec une masse par unité de surface de 10 à 1.500 g/m² et avec une surface efficace prédéterminée, qui donne une capacité de dénitrification optimale dans l'étage d'épuration respectif pour un débit donné par unité de surface et de temps, constitué d'au moins 50 % en poids de filaments continus de poly-ε-caprolactone, le polycaprolactone présentant en moyenne une masse moléculaire de 20.000 à 70.000 et les filaments individuels adhérant ensemble aux points de croisement sans agents de liaison.

2. Matériau support selon la revendication 1, caractérisé en ce que les filaments continus sont constitués entièrement de poly-ε-caprolactone.

3. Matériau support selon la revendication 1, caractérisé en ce que les filaments continus sont constitués d'un mélange polymère bicomposant, un des composants étant le polycaprolactone, l'autre étant un copolymère biodégradable de polyhydroxybutyrate-hydroxyvalérate, un polylactide ou un polyesteruréthane.
